# EUROPEAN PATENT APPLICATION

(11) **EP 4 108 343 A1**
(43) Date of publication of application: **28.12.2022**
(21) Application number: 20919591.6
(22) Date of filing: 08.12.2020
(51) Int. Cl.: B05B 5/025, A45D 19/16, A61Q 5/00, B05D 1/04, B05D 7/24

(54) **COMPOSITION FOR ELECTROSTATIC SPRAYING AND ELECTROSTATIC SPRAYING DEVICE**

(30) Priority: 21.02.2020 JP 2020028773
(71) Applicant: Panasonic Intellectual Property Management Co., Ltd., Osaka-shi, Osaka 540-6207 (JP)
(72) Inventor: KINOSHITA, Masato, Osaka-shi, Osaka 540-6207 (JP); OIKAZE, Hirotoshi, Osaka-shi, Osaka 540-6207 (JP); CHIKAZAWA, Yuki, Osaka-shi, Osaka 540-6207 (JP); INOUE, Hiroyuki, Osaka-shi, Osaka 540-6207 (JP); HANATO, Yumi, Osaka-shi, Osaka 540-6207 (JP)
(74) Representative: Grünecker Patent- und Rechtsanwälte PartG mbB
(86) International application number: PCT/JP2020/045585
(87) International publication number: WO 2021/166385

(57) **Abstract**

A composition for electrostatic spraying according to the present disclosure contains a microcapsule, a solvent for dispersion, and an acidic conductive regulator. An electrostatic spraying device includes an atomizer that electrostatically atomizes the composition for electrostatic spraying. Since the composition for electrostatic spraying contains an acidic conductive regulator, droplets that are acidic aqueous solutions are generated by electrostatic atomization. When the generated droplets are supplied to a supply target site, an effect based on a property of the droplets as the acidic aqueous solutions is exerted on the supply target site.

## Description

### TECHNICAL FIELD

The present disclosure relates to a composition for electrostatic spraying and an electrostatic spraying device.

### BACKGROUND ART

A composition for electrostatic spraying contains microcapsules. The microcapsule includes a core substance that exerts an effect on a target site and a wall substance containing the core substance. The composition for electrostatic spraying is electrostatically atomized by an electrostatic spraying device. When the composition for electrostatic spraying is electrostatically atomized, droplets are generated. The generated droplets are supplied to a supply target site. When the droplet containing the microcapsule is supplied to the supply target site and the wall substance is broken, the core substance is released, and the core substance is supplied to the supply target site.

As a composition for electrostatic spraying containing a microcapsule, a solvent for dispersion, and a conductive regulator, a composition for electrostatic spraying described in, for example, PTL 1 is known. This composition for electrostatic spraying contains a microcapsule containing a core substance that cares for hair and a wall substance having a cationic property fit with hair, a solvent for dispersion such as water, and a conductive regulator having a surfactant.

### Citation List

### Patent Literature

PTL 1: Unexamined Japanese Patent Publication No. 2018-176047

### SUMMARY OF THE INVENTION

It may take time until the microcapsule supplied to the supply target site exerts an effect. It is preferable that the effect be quickly exerted on the supply target site.

A composition for electrostatic spraying of the present disclosure contains a microcapsule, a solvent for dispersion, and an acidic conductive regulator.

According to the present disclosure, a preferred effect can be exerted on a supply target site.

### BRIEF DESCRIPTION OF THE DRAWINGS

Fig. 1 is a view illustrating one example of a model of an electrostatic spraying device.
Fig. 2 is a view illustrating one example of a model of a microcapsule.
Fig. 3 is a table showing conditions and results of tests.

### DESCRIPTION OF EMBODIMENT

(Exemplary forms of composition for electrostatic spraying and the like)

A composition for electrostatic spraying contains a microcapsule, a solvent for dispersion, and an acidic conductive regulator.

The composition for electrostatic spraying is electrostatically atomized by an electrostatic spraying device. Since the composition for electrostatic spraying contains an acidic conductive regulator, droplets that are acidic aqueous solutions are generated by electrostatic atomization. When the generated droplets are supplied to a supply target site, an effect based on a property of the droplets as the acidic aqueous solutions is exerted on the supply target site.

In one example of the composition for electrostatic spraying, a core substance of the microcapsule contains a component that exerts an effect on head hair.

The composition for electrostatic spraying contributes to head hair care.

An electrostatic spraying device includes an atomizer that electrostatically atomizes a composition for electrostatic spraying containing a microcapsule, a solvent for dispersion, and a conductive regulator, in which the atomizer includes an atomization electrode, a counter electrode having an opening, and a scattering suppressor disposed on an opposite side to the atomization electrode with respect to the counter electrode and disposed at a position lower than the opening.

The droplets generated by electrostatic atomization may include droplets each having a large particle size. The droplets each having a large particle size easily move downward by their own weights. According to the electrostatic spraying device, the droplets each having a large particle size may come into contact with the scattering suppressor. The droplet that has come in contact with the scattering suppressor is not supplied to the supply target site. The droplet having a large particle size is suppressed from being supplied to the supply target site.

An electrostatic spraying device includes an atomizer that electrostatically atomizes a composition for electrostatic spraying containing a microcapsule, a solvent for dispersion, and a conductive regulator, in which the atomizer electrostatically atomizes the composition for electrostatic spraying such that a peak, regarding a particle size distribution of droplets each not containing the microcapsule among the droplets generated from the composition for electrostatic spraying by the electrostatic atomization, is included in a range of 1 nm to 100 nm inclusive.

The droplet generated by the electrostatic spraying device easily enter a fine portion of the supply target site. This contributes to enhancing an effect of the droplet. The fine portion of the supply target site is, for example, a gap between cell membrane complexes of head hair when the supply target site is a head.

In one example of the electrostatic spraying device, the conductive regulator is acidic.

When the electrostatic spraying device electrostatically atomizes the composition for electrostatic spraying without using corona discharge, droplets that are acidic aqueous solutions are generated. When the generated droplets are supplied to a supply target site, an effect based on a property of the droplets as the acidic aqueous solutions is exerted on the supply target site. When corona discharge is not used for the electrostatic atomization, the microcapsules can be suppressed from being broken or modified during the electrostatic atomization.

### (First exemplary embodiment)

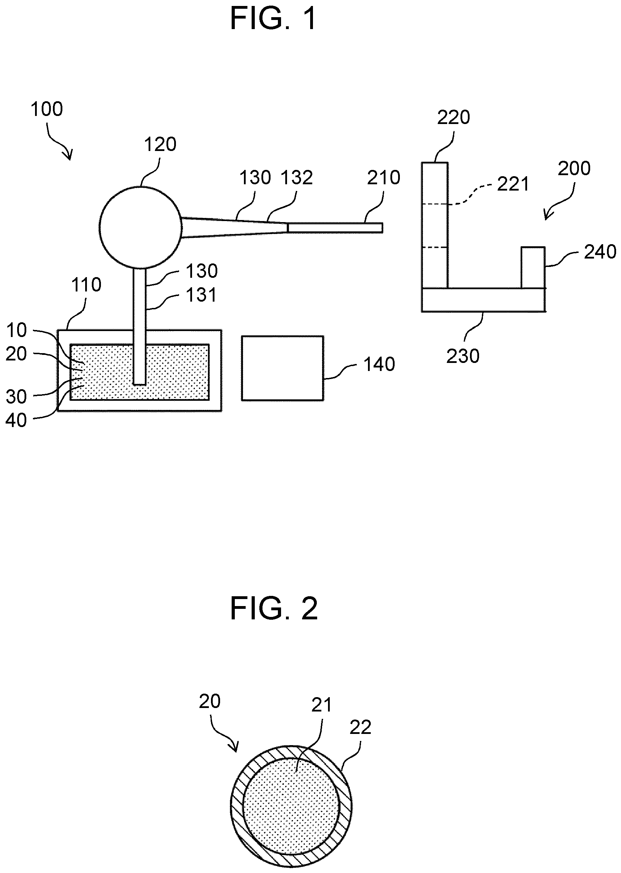

Fig. 1 illustrates one example of electrostatic spraying device 100. Electrostatic spraying device 100 electrostatically atomizes composition for electrostatic spraying 10.

Composition for electrostatic spraying 10 contains microcapsule 20, solvent for dispersion 30, and conductive regulator 40 that is acidic. When composition for electrostatic spraying 10 is electrostatically atomized, droplets are generated. The generated droplets are supplied to a supply target site. The supply target site is a portion on which an effect is exerted by an acidic component and the like. The supply target site is, for example, a predetermined site of a living body. The predetermined site of a living body is, for example, a head.

As illustrated in Fig. 2, microcapsule 20 has core substance 21 and wall substance 22. Core substance 21 contains a component that exerts an effect on the supply target site. When the supply target site is a head, microcapsule 20 contains core substance 21 that exerts an effect on head hair. Core substance 21 has, for example, a sustained release property. Wall substance 22 contains core substance 21. When the droplet containing microcapsule 20 is supplied to the supply target site and wall substance 22 is broken, core substance 21 is released, and core substance 21 is supplied to the supply target site. Wall substance 22 is broken, for example, by a lapse of time or physical stimulation. When the supply target site is a head, the physical stimulation is applied to wall substance 22 by contact with a hand, a comb, or the like, so that wall substance 22 is broken.

Core substance 21 contains an anionic hydrophobic substance. Examples of a material of core substance 21 include: oils and fats such as avocado oil, olive oil, cocoa butter, beef tallow, wheat germ oil, sansanqua oil, safflower oil, soybean oil, tea seed oil, evening primrose oil, camellia oil, tea tree oil, palm kernel oil, palm oil, castor oil, sunflower oil, macadamia nut oil, manuka oil, horse fat, cottonseed oil, Japan wax, moringa oil, and coconut oil; silicones such as methylpolysiloxane, methylphenylpolysiloxane, cyclic dimethyl silicone oil, alkyl modified silicone, amino modified silicone, and three-dimensional network structure silicone; higher fatty acids such as lauric acid, myristic acid, palmitic acid, stearic acid, isostearic acid, behenic acid, oleic acid, linoleic acid, and linolenic acid; waxes such as carnauba wax, candelilla wax, jojoba oil, beeswax, and lanolin; vitamins; vitamin-like active substances; and essential oils.

Core substance 21 is made of, for example, one kind or a combination of two or more kinds of materials selected from the materials described above as examples.

Wall substance 22 contains a cationic polymer. In a preferred example, the cationic polymer is a cationic polysaccharide-based polymer. Examples of the material of wall substance 22 include chitosan, gelatin, polyethyleneimine, agarose, β-lactoglobulin, poly-L-lysine, and polyarginine.

Wall substance 22 is made of, for example, one kind or a combination of two or more kinds of materials selected from the materials described above as examples.

Solvent for dispersion 30 disperses microcapsules 20. Solvent for dispersion 30 becomes a raw material of the droplets generated by the electrostatic atomization. Solvent for dispersion 30 is, for example, a 1.0 wt% sodium chloride aqueous solution. An additive may be added to solvent for dispersion 30. The additive is, for example, a salt, an alcohol, or the like.

Conductive regulator 40 makes a pH of the composition for electrostatic spraying acidic. Examples of conductive regulator 40 include a fatty acid, an alkanoic acid (linear saturated fatty acid), an alkynoic acid, a polyunsaturated fatty acid, an α-hydroxy acid, a dicarboxylic acid, an acidic amino acid, and an inorganic acid.

Examples of the fatty acid include lauric acid, myristic acid, palmitic acid, stearic acid, behenic acid, oleic acid, isostearic acid, oxystearic acid, and undecylenic acid.

Examples of the alkanoic acid include formic acid, acetic acid, propionic acid, butyric acid, valeric acid, caproic acid, enanthic acid, caprylic acid, pelargonic acid, capric acid, and undecanoic acid.

Examples of the alkynoic acid include: linear alkenoic acids such as acrylic acid, crotonic acid, isocrotonic acid, acetovylic acid, β-ethyl acrylic acid, and acetoallylic acid; propynoic acid; tetrolic acid; 2-pentynic acid; allylacetic acid; 2-hexynic acid; 2-heptinic acid; 2-octynic acid; 2-nonic acid; and 2-decinic acid.

Examples of the polyunsaturated fatty acid include sorbic acid, linoleic acid, inoelaidic acid, α-linolenic acid, γ-linolenic acid, punicaic acid, stearidonic acid, EPA, DHA, sardine acid, herring acid, and arachidonic acid.

Examples of the α-hydroxy acid include citric acid, glycolic acid, tartaric acid, lactic acid, malic acid, and ascorbic acid.

Examples of the dicarboxylic acid include oxalic acid, malonic acid, succinic acid, glutalic acid, adipic acid, pimelic acid, fumaric acid, maleic acid, phthalic acid, isophthalic acid, and terephthalic acid.

Examples of the acidic amino acid include glutamic acid and aspartic acid.

Examples of the inorganic acid include phosphoric acid and nitric acid.

Conductive regulator 40 is made of, for example, one kind or a combination of two or more kinds of materials selected from the materials described above as examples.

One example of a method for manufacturing composition for electrostatic spraying 10 will be described.

A first step is a step of preparing an oil-in-water emulsion solution. In the first step, core substance 21, or an anionic hydrophobic substance, is dispersed in an aqueous phase. When core substance 21 is dispersed in the aqueous phase, the oil-in-water emulsion solution (o/w solution) of core substance 21 is generated. For the dispersion of core substance 21, an emulsifying device or a homogenizer, for example, is used. When a surfactant suitable for the dispersion of core substance 21 is added to the aqueous phase, dispersibility of core substance 21 is enhanced. Core substance 21 is, for example, camellia oil. The aqueous phase of the oil-in-water emulsion solution of core substance 21 is, for example, distilled water, an aqueous solution with a pH between 4 and 7 (inclusive), or an aqueous solution containing a lower alcohol with less than or equal to five carbon atoms. Examples of the lower alcohol with less than or equal to five carbon atoms include glycerin, ethanol, and isopropanol.

A content of the lower alcohol with less than or equal to five carbon atoms (hereinafter referred to as "lower alcohol content") affects solubility of each component contained in composition for electrostatic spraying 10. In a preferred example, the lower alcohol content is determined such that the solubility of each component contained in composition for electrostatic spraying 10 is improved. In a first example, the lower alcohol content is more than 0% by mass and less than or equal to 45% by mass. In a second example, the lower alcohol content is more than 0% by mass and less than or equal to 30% by mass. In a third example, the lower alcohol content is more than 0% by mass and less than or equal to 20% by mass. In a fourth example, the lower alcohol content is more than 0% by mass and less than or equal to 15% by mass. In a fifth example, the lower alcohol content is more than 0% by mass and less than or equal to 10% by mass.

A second step is a step of adding the fatty acid. In the second step, the fatty acid with more than or equal to five carbon atoms is added to the oil-in-water emulsion solution. An anionic strength of core substance 21 is adjusted by adding the fatty acid. In one example, when a weight percentage of the fatty acid with more than or equal to five carbon atoms contained in core substance 21 is less than 80%, the fatty acid is added such that the weight percentage is more than or equal to 80%. When the weight percentage is more than or equal to 80%, microcapsule 20 having a sufficient film thickness is easily generated. Examples of the fatty acid with more than or equal to five carbon atoms include hexanoic acid, heptanoic acid, octanoic acid, lauric acid, stearic acid, linoleic acid, and oleic acid. The fatty acid to be added is made of, for example, one kind or a combination of two or more kinds of materials selected from the materials described above as examples.

A step of adjusting particle size may be added to the second step. In the step of adjusting particle size, a particle size of core substance 21 in the oil-in-water emulsion solution is adjusted. With the particle size of core substance 21 adjusted, a particle size of microcapsule 20 finally generated can be adjusted.

A third step is an inclusion step. In the third step, while the oil-in-water emulsion solution is being stirred, an aqueous solution of wall substance 22, or a cationic polymer, is added and mixed. The aqueous solution is, for example, a dilute acid solution of chitosan. A particle dispersion liquid is separated from the mixed aqueous solution using a separatory funnel. Microcapsules 20 are dispersed in the separated particle dispersion liquid.

A fourth step is a step of adding a regulator. In the fourth step, conductive regulator 40 that is acidic is added to the particle dispersion liquid to adjust a conductivity (electrical conductivity) of the aqueous phase of composition for electrostatic spraying 10. Conductive regulator 40 that is acidic is, for example, sodium nitrate. Through the fourth step, composition for electrostatic spraying 10 containing microcapsule 20, solvent for dispersion 30, and conductive regulator 40 that is acidic, is generated.

As illustrated in Fig. 1, electrostatic spraying device 100 includes atomizer 200. Atomizer 200 electrostatically atomizes composition for electrostatic spraying 10. Atomizer 200 includes atomization electrode 210, counter electrode 220, support 230, and scattering suppressor 240. Atomization electrode 210 applies a potential to composition for electrostatic spraying 10 and electrostatically atomizes composition for electrostatic spraying 10. A material of atomization electrode 210 is, for example, stainless steel. Atomization electrode 210 has, for example, a cylindrical shape. An inner space of atomization electrode 210 makes up a flow path through which composition for electrostatic spraying 10 flows. The inner space of atomization electrode 210 opens at a tip of atomization electrode 210. An inner diameter of atomization electrode 210 is determined such that even when corona discharge is not used, composition for electrostatic spraying 10 is electrostatically atomized. The inner diameter of atomization electrode 210 is smaller than an inner diameter of the atomization electrode on the premise of using corona discharge. The inner diameter of atomization electrode 210 is, for example, 60 µm. An outer diameter of atomization electrode 210 is, for example, 100 µm.

Counter electrode 220 is a ground for atomization electrode 210. A material of counter electrode 220 is, for example, stainless steel. Counter electrode 220 is, for example, a thin plate. Counter electrode 220 has, for example, an annular shape. Counter electrode 220 having an annular shape includes opening 221. A central axis of opening 221 is coaxial with a central axis of the annular shape. An inner diameter of counter electrode 220 having an annular shape is determined in consideration of, for example, an intensity of an electric field acting on a Taylor cone formed at the tip of atomization electrode 210 and a strength of an adsorption force of counter electrode 220 acting on the droplet generated by splitting of the Taylor cone. The inner diameter of counter electrode 220 is, for example, 2 mm.

Support 230 supports counter electrode 220. Support 230 has an electrical insulation property. Support 230 is provided, for example, in a housing of electrostatic spraying device 100. Counter electrode 220 is fixed to support 230. In one example, atomization electrode 210 and counter electrode 220 are disposed such that a central axis of atomization electrode 210 and a central axis of counter electrode 220 are coaxial. A gap between atomization electrode 210 and counter electrode 220 is determined in consideration of, for example, a size of electrostatic spraying device 100. The gap between atomization electrode 210 and counter electrode 220 is, for example, 5 mm.

When composition for electrostatic spraying 10 is electrostatically atomized by electrostatic spraying device 100, droplets are generated. In terms of the size of particle size, the generated droplets can be classified into, for example, droplets each having a small particle size and droplets each having a large particle size. The droplets each having a small particle size easily pass through the opening of counter electrode 220. The droplets that have passed through the opening of counter electrode 220 are supplied to the supply target site.

Scattering suppressor 240 suppresses the droplets each having a large particle size from being supplied to the supply target site. Scattering suppressor 240 is disposed on the opposite side to atomization electrode 210 with respect to counter electrode 220. Scattering suppressor 240 is provided downstream, in a traveling direction of the droplets, of counter electrode 220. Scattering suppressor 240 is provided, for example, in support 230. Scattering suppressor 240 is, for example, a wall. A structure of the wall is, for example, a flat plate. A material of scattering suppressor 240 is, for example, stainless steel. In one example, in a state where atomizer 200 is disposed such that the central axis of counter electrode 220 having an annular shape is parallel to the horizontal, scattering suppressor 240 is configured to be positioned below a lowest portion, in the vertical direction, of opening 221.

The droplets each having a large particle size easily move downward by their own weights. The droplets each having a large particle size may come into contact with scattering suppressor 240. The droplet that has come in contact with scattering suppressor 240 is not supplied to the supply target site. Atomizer 200 including scattering suppressor 240 suppresses the droplets each having a large particle size from being supplied to the supply target site.

In one example, electrostatic spraying device 100 further includes tank 110, pump 120, pipe 130, and controller 140. Tank 110 houses composition for electrostatic spraying 10. Tank 110 is, for example, a pouch. The pouch is made of, for example, a sheet. The sheet includes, for example, an aluminum foil and polyethylene.

Pump 120 supplies composition for electrostatic spraying 10 in tank 110 to atomization electrode 210. Pump 120 is provided between tank 110 and atomization electrode 210. Pump 120 is, for example, a gear pump.

Pipe 130 is configured to flow composition for electrostatic spraying 10. Pipe 130 includes, for example, first pipe 131 and second pipe 132. First pipe 131 connects tank 110 and pump 120. Second pipe 132 connects pump 120 and atomization electrode 210. When pump 120 operates, composition for electrostatic spraying 10 in tank 110 flows through first pipe 131 and second pipe 132 to be supplied to the inner space of atomization electrode 210.

Controller 140 controls pump 120 and atomizer 200. Controller 140 switches between a state where pump 120 operates and a state where the operation of pump 120 is stopped, according to operations of a switch or the like. Controller 140 switches between a state where a voltage is applied between atomization electrode 210 and counter electrode 220 and a state where no voltage is applied between atomization electrode 210 and counter electrode 220, according to operations of a switch or the like. In one example, a magnitude of the voltage to be applied between atomization electrode 210 and counter electrode 220 is predetermined. Controller 140 applies the predetermined voltage.

In terms of whether or not the droplets generated by atomizer 200 contain microcapsules 20, the droplets can be classified into droplets each containing microcapsule 20 (hereinafter referred to as "containing droplets") and droplets each not containing microcapsule 20 (hereinafter referred to as "non-containing droplets"). The containing droplet, the non-containing droplet, and microcapsule 20 are assumed to be spheres. A particle size of the containing droplet is more than or equal to the particle size of microcapsule 20. An average particle size of the containing droplets is, for example, 3 µm. In one example, atomizer 200 electrostatically atomizes composition for electrostatic spraying 10 such that a peak, regarding particle sizes of the non-containing droplets, is included in a range of 1 nm to 100 nm inclusive.

When electrostatic spraying device 100 is used for head hair care, composition for electrostatic spraying 10 in a preferred example is adjusted to have an acidity of pH 1 to pH 4. When fine droplets are generated from this composition for electrostatic spraying 10 and a small amount of the droplets, to an extent in which stickiness is not given to head hair, is supplied to head hair, the pH of the head hair easily approaches pH 5 that is an isoelectric point. This contributes to an improvement in touch feeling or the like when the head hair is dry. In addition, even when a spray amount of the droplets on head hair is small, a head hair care effect can be obtained. This effect contributes to downsizing tank 110 and increasing a continuous usable time.

One example of operation of electrostatic spraying device 100 will be described.

Composition for electrostatic spraying 10 is injected into tank 110. A power supply of electrostatic spraying device 100 is changed from off to on. The operation of electrostatic spraying device 100 is started. Controller 140 starts operation of pump 120. When pump 120 operates, composition for electrostatic spraying 10 is supplied to the inner space of atomization electrode 210. Controller 140 applies a DC voltage to atomization electrode 210 with respect to counter electrode 220. In a state where the voltage is applied, composition for electrostatic spraying 10 staying at the tip of atomization electrode 210 is charged, and the Taylor cone is generated. When the Taylor cone is split, droplets are generated. The generated droplets move toward counter electrode 220 according to a difference between potentials of atomization electrode 210 and counter electrode 220.

A state of the containing droplet may change as the containing droplet travels toward the supply target site. In one example, moisture of the containing droplet evaporates as the containing droplet travels toward the supply target site. When the moisture of the containing droplet almost evaporates at a stage where the containing droplet reaches the supply target site, components that make up the containing droplet are approximately microcapsules 20. Wall substance 22 of microcapsule 20 attached to the supply target site is broken by a lapse of time or physical stimulation. As wall substance 22 is broken, core substance 21 is released. In one example, the supply target region is a head, and core substance 21 is camellia oil. The droplets are supplied to the head by electrostatic spraying device 100, and microcapsules 20 are attached to the head hair. As wall substance 22 is broken, camellia oil that is core substance 21 is released to the head hair, and a hair care effect based on a property of the camellia oil is exerted on the head hair.

A state of the non-containing droplet may change as the non-containing droplet travels toward the supply target site. In one example, moisture of the non-containing droplet evaporates as the non-containing droplet travels toward the supply target site. When the moisture of the non-containing droplet almost evaporates at a stage where the non-containing droplet reaches the supply target site, components that make up the non-containing droplet are approximately nitric acid hydrate or nitrate. An effect based on a property of the non-containing droplet attached to the supply target site is exerted on the supply target site immediately after the attachment of the non-containing droplet. When the non-containing droplet is attached to head hair, a head hair care effect based on the property of the non-containing droplet is exerted on the head hair.

The properties of conventional compositions for electrostatic spraying and substances related thereto will be studied. Microcapsules have an excellent long-lasting effect. There is room for improvement in an immediate effect of the microcapsules. Examples of a component having a high immediate effect include an acidic aqueous solution. When water is electrostatically atomized by corona discharge, an acidic aqueous solution that is a droplet containing a nitric acid component is generated. A technique related to this is described in, for example, Unexamined Japanese Patent Publication No. 2011-5267.

It is also considered that when a composition for electrostatic spraying containing a microcapsule is electrostatically atomized by corona discharge to generate a microcapsule-containing droplet, the droplet achieves both the immediate effect and long-lasting effect. However, when the composition for electrostatic spraying is electrostatically atomized by corona discharge, there is a risk that the microcapsule may be broken or modified, and the effect of the microcapsule may be decreased. If the droplet, containing the microcapsule that has been broken or modified, is supplied to the supply target site, it is considered that the effect of the microcapsule is not sufficiently exerted on the supply target site.

Examples of a method for suppressing breakage and modification of the microcapsule during the electrostatic atomization include a method for electrostatically atomizing a composition for electrostatic spraying without using corona discharge. In this method, the acidic aqueous solution that is a droplet containing a nitric acid component is not generated, unlike the case where the composition for electrostatic spraying is electrostatically atomized using corona discharge. Since the droplet generated by the electrostatic atomization is not an acidic aqueous solution, it is difficult to obtain a high immediate effect when the droplet is supplied to the supply target site.

Actions and effects of composition for electrostatic spraying 10 and electrostatic spraying device 100 of a first exemplary embodiment will be described. Composition for electrostatic spraying 10 contains conductive regulator 40 that is acidic. When composition for electrostatic spraying 10 is electrostatically atomized by electrostatic spraying device 100, a droplet that is an acidic aqueous solution is generated. This droplet has a high immediate effect. Electrostatic spraying device 100 electrostatically atomizes composition for electrostatic spraying 10 without using corona discharge. Breakage and modification of microcapsule 20 during the electrostatic atomization are suppressed. Microcapsule 20 contained in the droplet generated by the electrostatic atomization has desired properties. Microcapsule 20 has a high long-lasting effect. When the droplet having the property of an acidic aqueous solution is supplied to the supply target site, the effect of the droplet is immediately exerted on the supply target site due to the properties of the droplet as the acidic aqueous solution. When the containing droplet is supplied to the supply target site and wall substance 22 is broken, the effect of core substance 21 is continuously exerted on the supply target site.

### (Examples)

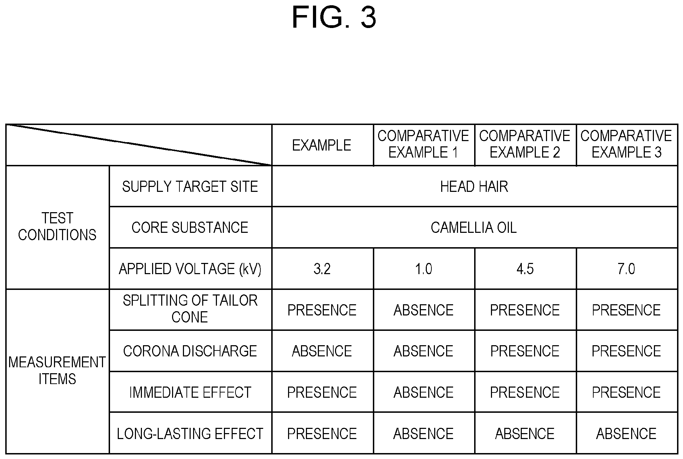

With reference to Fig. 3, tests on the effects of the composition for electrostatic spraying will be described. Subjects of these tests are an example and comparative examples 1 to 3. An electrostatic spraying device in each of the example and the comparative examples is electrostatic spraying device 100 of the first exemplary embodiment. A supply target site in each of the example and the comparative examples is head hair. A core substance of a microcapsule in each of the example and the comparative examples is camellia oil.

Voltages (hereinafter referred to as "applied voltages") applied between the atomization electrode and the counter electrode are at four levels. The applied voltage in the example is 3.2 kV. The applied voltage in the comparative example 1 is 1.0 kV. The applied voltage in the comparative example 2 is 4.5 kV. The applied voltage in the comparative example 3 is 7.0 kV.

Four items are measured. A first measurement item is whether or not the Taylor cone was split. A second measurement item is whether or not corona discharge occurred. A measurement method for each of the first and second measurement items is visual measurement by testers. When corona discharge occurs between the atomization electrode and the counter electrode, the Taylor cone is split due to an influence of the corona discharge. Whether or not corona discharge occurs is considered to be affected by, for example, the structure of the electrostatic spraying device, the conductivity of the composition for electrostatic spraying, and the characteristics of the applied voltage.

A third measurement item is whether or not the immediate effect related to the head hair care could be felt when the electrostatic spraying device was used. A fourth measurement item is whether or not the long-lasting effect related to the head hair care could be felt when the electrostatic spraying device was used. A measurement method for each of the third and fourth measurement items is sensory evaluation by subjects. There are ten subjects.

"Presence" described in the column of the first measurement item in the table indicates a case where it was confirmed by the visual measurement that the Taylor cone was split. "Absence" described in the same column indicates a case where it was not confirmed by the visual measurement that the Taylor cone was split. "Presence" described in the column of the second measurement item in the table indicates a case where it was confirmed by the visual measurement that corona discharge occurred. "Absence" described in the same column indicates a case where it was not confirmed by the visual measurement that corona discharge occurred. "Presence" described in the column of the third measurement item in the table indicates a case where the immediate effect related to the head hair care could be felt by the sensory evaluation. "Absence" described in the same column indicates a case where the immediate effect related to the head hair care could not be felt by the sensory evaluation. "Presence" described in the column of the fourth measurement item in the table indicates a case where the long-lasting effect related to the head hair care could be felt by the sensory evaluation. "Absence" described in the same column indicates a case where the long-lasting effect related to the head hair care could not be felt by the sensory evaluation.

In the example, fine droplets were generated. This is considered to be affected by the magnitude of the applied voltage with respect to the inner diameter of the atomization electrode. In the comparative example 1, corona discharge did not occur, so that it is considered that no droplets were generated from the composition for electrostatic spraying, and no droplets were supplied to the head hair. In the comparative examples 2 and 3, it is considered that droplets were generated from the composition for electrostatic spraying due to occurrence of corona discharge, and the microcapsules were broken or modified during the electrostatic atomization.

### (Second exemplary embodiment)

Electrostatic spraying device 100 of a second exemplary embodiment is different, in a part of its configuration, from electrostatic spraying device 100 of the first exemplary embodiment. The other configurations are common. Electrostatic spraying device 100 of the first exemplary embodiment includes atomizer 200 that electrostatically atomizes composition for electrostatic spraying 10 without using corona discharge. Electrostatic spraying device 100 of the second exemplary embodiment includes atomizer 200 that electrostatically atomizes composition for electrostatic spraying 10 by corona discharge, and controller 140 that adjusts the intensity of the corona discharge.

Controller 140 controls the applied voltage such that corona discharge occurs between atomization electrode 210 and counter electrode 220. Controller 140 controls the applied voltage such that a breakage rate of microcapsules 20 during the electrostatic atomization is less than or equal to a predetermined value. The breakage of microcapsules 20 includes breakage and modification of microcapsules 20. The breakage rate of microcapsules 20 is determined, for example, from an amount of microcapsules 20 supplied to the supply target site and an amount of broken microcapsules 20 contained therein. A relationship between the breakage rate of microcapsules 20 and the applied voltage is set, for example, in advance. In one example, controller 140 controls the applied voltage by referring to data, indicating the relationship between the breakage rate of microcapsules 20 and the applied voltage, stored in a storage unit or the like of electrostatic spraying device 100.

### (Third exemplary embodiment)

Electrostatic spraying device 100 of a third exemplary embodiment is different, in a part of its configuration, from electrostatic spraying device 100 of the second exemplary embodiment. The other configurations are common. Controller 140 of the third exemplary embodiment controls the applied voltage based on a factor (hereinafter referred to as a "related factor") related to the immediate effect and the long-lasting effect of the droplets. The related factor includes, for example, at least one of the property of core substance 21 of microcapsule 20, a user's preference regarding head hair condition, and when to use electrostatic spraying device 100 by the user. When to use electrostatic spraying device 100 is set based on, for example, time and activities of daily life. Examples of the activities of daily life include before going out, after bathing, and before going to bed. A relationship between the related factor and the applied voltage is set, for example, in advance. In one example, the controller 140 controls the applied voltage by referring to data, indicating the relationship between the related factor and the applied voltage, stored in a storage unit or the like of electrostatic spraying device 100. Controller 140 controls the applied voltage according to the related factor such that the immediate effect and long-lasting effect of the droplet are both achieved. This contributes to realizing an immediate effect and a long-lasting effect that are expected by, for example, a user.

### (Fourth exemplary embodiment)

Electrostatic spraying device 100 of a fourth exemplary embodiment includes the configurations of both the second exemplary embodiment and the third exemplary embodiment. Controller 140 controls the applied voltage based on the breakage rate of microcapsules 20 and the related factor.

### (Fifth exemplary embodiment)

Electrostatic spraying device 100 of a fifth exemplary embodiment is different, in a part of its configuration, from electrostatic spraying device 100 of each of the first to fourth exemplary embodiments. The other configurations are common. Electrostatic spraying device 100 of the fifth exemplary embodiment further includes a supply promoter. The supply promoter has a function of promoting supply of the droplets to the supply target site. The supply promoter includes, for example, an air blower.

### (Sixth exemplary embodiment)

Electrostatic spraying device 100 of a sixth exemplary embodiment is different, in a part of its configuration, from electrostatic spraying device 100 of each of the first to fifth exemplary embodiments. The other configurations are common. Electrostatic spraying device 100 of the sixth exemplary embodiment is configured to be able to be incorporated in a main device. The main device is, for example, an electrical appliance to be used at home or the like. Examples of the main device include an air conditioner, a bathroom ventilation dryer, a washing machine, a washing and drying machine, a futon dryer, a clothes drying dehumidifier, a refrigerator, a toilet seat, a dishwasher, an air cleaner, a humidifier, a fan heater, an electric fan, a deodorizing hanger, a shoe deodorizer, a steamer, and a hair dryer. A material of composition for electrostatic spraying 10 is selected according to the application of the main device.

The controller of each of the exemplary embodiments described above includes, for example, a microcontroller having one or more processors and one or more memories. The microcontroller realizes a function as the controller by executing programs recorded in one or more memories by one or more processors. The programs may be recorded in the memories in advance, may be provided by being recorded in a non-transitory recording medium such as a memory card, or may be provided through an electric communication line. In other words, the programs are ones for making one or more processors function as the controller.

The description of each of the exemplary embodiments described above is not intended to limit forms that can be taken by the composition for electrostatic spraying and the electrostatic spraying device according to the present disclosure. The composition for electrostatic spraying and the electrostatic spraying device according to the present disclosure can take forms different from the forms described as examples in each of the exemplary embodiments. One example thereof is a form in which a part of the configuration of each of the exemplary embodiments is replaced, changed, or omitted, or a form in which a new configuration is added to each of the exemplary embodiments.

The composition for electrostatic spraying and the electrostatic spraying device of the present disclosure can be used for head hair care and the like.

### REFERENCE MARKS IN THE DRAWINGS

- 10: composition for electrostatic spraying
- 20: microcapsule
- 21: core substance
- 22: wall substance
- 30: solvent for dispersion
- 40: conductive regulator
- 100: electrostatic spraying device
- 110: tank
- 120: pump
- 130: pipe
- 131: first pipe
- 132: second pipe
- 140: controller
- 200: atomizer
- 210: atomization electrode
- 220: counter electrode
- 221: opening
- 230: support
- 240: scattering suppressor

## Claims

1. A composition for electrostatic spraying, the composition comprising:
a microcapsule;
a solvent for dispersion; and
an acidic conductive regulator.

2. The composition according to Claim 1, wherein a core substance of the microcapsule contains a component that exerts an effect on head hair.

3. An electrostatic spraying device comprising an atomizer that electrostatically atomizes a composition for electrostatic spraying, the composition containing a microcapsule, a solvent for dispersion, and a conductive regulator,
wherein the atomizer includes:
an atomization electrode;
a counter electrode having an opening; and
a scattering suppressor disposed on an opposite side to the atomization electrode with respect to the counter electrode and disposed at a position lower than the opening.

4. An electrostatic spraying device comprising an atomizer that electrostatically atomizes a composition for electrostatic spraying, the composition containing a microcapsule, a solvent for dispersion, and a conductive regulator,
wherein the atomizer electrostatically atomizes the composition for electrostatic spraying to make a peak regarding a particle size distribution of droplets be included in a range of 1 nm to 100 nm inclusive, the droplets each not containing the microcapsule among droplets generated from the composition for electrostatic spraying by electrostatic atomization.

5. The electrostatic spraying device according to Claim 3 or Claim 4, wherein the conductive regulator is acidic.
